# EUROPÄISCHE PATENTSCHRIFT

(11) **EP 2 736 602 B1**
(45) Veröffentlichungstag und Bekanntmachung des Hinweises auf die Patenterteilung: **24.04.2019**
(21) Anmeldenummer: 12723713.9
(22) Anmeldetag: 29.05.2012
(51) Int. Cl.: A61K 8/44, A61K 8/41, A61K 8/04, A61K 8/22, A61Q 5/08

(54) **AUFHELLVERFAHREN MIT BLONDIERMOUSSE**
LIGHTENING PROCESS WITH BLONDING MOUSSE
PROCÉDÉ D'ÉCLAIRCISSEMENT AVEC MOUSSE À BLONDIR

(30) Priorität: 27.07.2011 DE 102011079922
(43) Veröffentlichungstag der Anmeldung: 04.06.2014
(73) Patentinhaber: Henkel AG & Co. KGaA, 40589 Düsseldorf (DE)
(72) Erfinder: SCHMAHL, Melanie, CH-4334 Sisseln (CH); JANßEN, Frank, 41470 Neuss (DE)
(86) Internationale Anmeldenummer: PCT/EP2012/060036
(87) Internationale Veröffentlichungsnummer: WO 2013/013862

(56) Entgegenhaltungen:
- EP-A2- 2 324 811
- DE-A1-102009 028 523
- DE-U1-202009 011 154
- US-B2- 7 505 261
- US-B2- 7 507 261

## Beschreibung

Die Erfindung betrifft Verfahren zur Aufhellung von keratinhaltigen Fasern, bei denen ein anwendungsbereites Mittel durch Vermischen von 3 Komponenten hergestellt und zur Anwendung aus einem Schaumspendergefäß ausgebracht wird sowie entsprechende Mittel und Mehrkomponentenverpackungseinheiten zur Aufhellung von keratinhaltigen Fasern.

Für das Aufhellen von keratinhaltigen Fasern kommen im allgemeinen oxidative Aufhellmittel zu Einsatz. Die enthaltenen Oxidationsmittel, zumeist zumindest Wasserstoffperoxid, dienen dabei dazu, natürliche Melanin-Farbstoffe sowie gegebenenfalls künstliche Farbstoffe in und auf der keratinischen Faser zu zerstören und so eine Entfärbung der Faser zu bewirken. Für die Aufhellung von dunklen Ausgangsfasern ist zumeist der Zusatz weiterer Oxidationsmittel, insbesondere von Persalzen, wie Persulfaten, notwendig.

Zumeist besitzen oxidative Aufhellmittel zur Reaktionsbeschleunigung während der oxidativen Anwendung einen alkalischen pH-Wert, der mit Alkalisierungsmitteln, wie Alkanolaminen, Ammoniak oder anorganischen Basen, eingestellt wird. Insbesondere Ammoniak ermöglicht dabei zwar gute Aufhellergebnisse, offenbart jedoch aufgrund seines Geruchs und Reizpotentials für Haut und Schleimhäute auch Nachteile für den Anwender. Daher liegen verstärkte Bemühungen auf der Entwicklung leistungsfähiger oxidativer Aufhellmittel, die auf den Einsatz von Ammoniak verzichten.

Oxidative Aufhellmittel bestehen üblicherweise aus zwei oder mehr Komponenten, deren Mischung ausreichend zähflüssig ist, damit sie bequem auf die Haare aufgetragen werden kann und dabei nicht tropft oder verläuft. Im Falle des Einsatzes von Persalzen ist diese Komponente zweckmäßigerweise pulverförmig. Dadurch ergeben sich jedoch weitere Herausforderungen an solche Aufhellmittel. Gerade die Herstellung der anwendungsbereiten Mittel durch Vermischen von pulverförmigen Blondiersalz-Komponenten und häufig hochviskosen, creme- oder pastenförmigen Alkalisierungszubereitungen und Entwicklerzubereitungen ist häufig aufwendig und mit Problemen einer gleichmäßigen, innigen Durchmischung behaftet. Daher müssen insbesondere eine einfache und schnelle Anmischbarkeit und eine gute Auftragbarkeit der Mittel gewährleistet sein, um Inhomogenitäten in Konzentration der Inhaltsstoffe während der Anwendung zu verhindern und so für ein gleichmäßiges, die Fasern möglichst wenig schädigendes Aufhellergebnis zu sorgen.

Aufgabe der vorliegenden Erfindung war es daher, Verfahren mit oxidativen Aufhellmitteln zu optimieren, so dass die oben genannten Nachteile überwunden werden können. Insbesondere sollten stabile Aufhellmittel ohne unangenehme Gerüche, die möglichst auch ammoniakfrei sein sollen, bereit gestellt werden.

Eine aus dem Bereich der oxidativen Haarfärbemittel bekannte Anwendungsform ist die Schaumapplikation, mit der der Verbraucher zumeist haarfaserschonende, gleichmäßige Färbeergebnisse assoziiert. Nachteilig für die Schaumapplikation ist jedoch der üblicherweise zur Schaumbildung genutzte Treibgaseinsatz, denn in jüngerer Zeit besteht das vermehrte Bedürfnis, auf den Einsatz von Treibgasen verzichten zu können.

Ein weiteres Problem bei der Schaumapplikation ist die Stabilisierung der Schäume. Die Beschaffenheit von Schäumen ist dann als ideal zu bezeichnen, wenn bei der Produktabgabe ein fester, stabiler Schaum entsteht, der ein geschmeidiges Gefühl hinterlässt und auf dem Haar nur langsam bricht. Häufig ist aber zu beobachten, dass die ausgebrachten Schäume wenig stabil sind und schnell wieder in sich zusammenfallen, so dass eine dünnflüssige, tropfende Lösung zurückbleibt. Andererseits ist es auch wesentlich, dass der Schaum trotzdem die Haare gut benetzt, so dass ein guter Aufhellungsprozess stattfinden kann. Die Schaumstabilität wird insbesondere durch die Anwesenheit größerer Mengen an Salzen negativ beeinflusst.

US 7 507 261 B2 betrifft Zusammensetzungen zum Färben von Haaren, welche durch Vermischen von mindestens zwei Komponenten hergestellt werden. DE 20 2009 011 154 U1 beschreibt enzymatisch gestützte Färbe- und Aufhellmittel. EP 2 324 811 beschreibt Färbemittel, die in Form von stabilen Schäumen vorliegen.

Aufgabe der vorliegenden Erfindung war es daher weiterhin, Verfahren für die Anwendung von oxidativen Aufhellmitteln für die Schaumapplikation ohne Verwendung von Treibgasen zu optimieren, so dass die oben genannten Nachteile überwunden werden können.
Es wurde überraschenderweise gefunden, dass sich oxidative Aufhellmittel in Form von stabilen Schäumen sehr leicht von Anwender herstellen und applizieren lassen und andererseits zu verbesserten Aufhellergebnissen führen.

Ein erster Gegenstand der vorliegenden Erfindung ist daher ein Verfahren zur Aufhellung keratinischer Fasern, welches die Schritte umfasst:
i. Herstellung des anwendungsbereiten Aufhellmittels unmittelbar vor der Anwendung durch Zusammenführen
   eines Mittels (A), enthaltend mindestens ein Alkalisierungsmittel,
   eines Mittels (B), enthaltend mindestens ein Oxidationsmittel, und
   eines Mittels (C), enthaltend mindestens ein Peroxosalz,
   und anschließendes Vermischen,
ii. Ausbringung des anwendungsbereiten Aufhellmittels aus einem Schaumspendergefäß und Verteilung des anwendungsbereiten Mittel auf den Fasern,
iii. Verbleib des Aufhellmittels für einen Zeitraum von 1 bis 60 min auf den Fasern
iv. und Auswaschen des verbliebenen Aufhellmittels aus den Fasern.
dadurch gekennzeichnet, dass das Mittel (A) zusätzlich mindestens ein zwitterionisches Tensid der Formel (I), 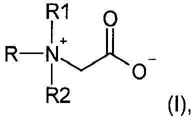 worin
- R: für eine gesättigte oder ungesättigte C₁₀-C₂₀-Alkylkette steht und
- R1 und R2: jeweils unabhängig voneinander für eine C₁-C₄-Alkylgruppe oder eine C₂-C₄-Hydroxyalkylgruppe stehen,
enthält.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen, anwendungsbereiten Aufhellmittel enthalten die Wirkstoffe in einem kosmetisch akzeptablen Träger. Dieser kosmetische Träger ist bevorzugt wässrig, alkoholisch oder wässrig-alkoholisch. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten, sofern sie die Schaumbildung und -stabilität nicht übermäßig negativ beeinflussen. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.
Das Mittel (A) enthält als erste erfindungswesentliche Komponente ein Alkalisierungsmittel. Die verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak.

Erfindungsgemäße, anorganische Alkalisierungsmittel werden bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt. Schließlich ist ein weiteres bevorzugtes Alkalisierungsmittel Ammoniak. Bevorzugt werden die weiteren Alkalisierungsmittel in einer Menge von 0,05 bis 10 Gew.-%, insbesondere von 0,5 bis 5 Gew.-%, jeweils bezogen auf das Gesamtgewicht des anwendungsbereiten Mittels, enthalten.

In einer besonders bevorzugten Ausführungsform werden die erfindungsgemäßen Mittel dabei frei von Ammoniak als Alkalisierungsmittel formuliert. "Frei von Ammoniak" bedeutet dabei erfindungsgemäß, dass der Ammoniakgehalt der erfindungsgemäßen Mittel unterhalb von 1 Gew.-%, vorzugsweise unterhalb von 0,5 Gew.-%, insbesondere unterhalb von 0,1 Gew.-%, jeweils bezogen auf das anwendungsbereite Mittel, liegt.

Es hat sich gezeigt, dass insbesondere die Verwendung von mindestens einem Alkanolamin als Alkalisierungsmittel positiv auf die Aufhellleistung der Mittel auswirkt und insbesondere erlaubt auf Ammoniak zu verzichten.

In einer bevorzugten Ausführungsform des ersten Erfindungsgegenstands enthält das Mittel (A) als Alkalisierungsmittel mindestens ein Alkanolamin.

Erfindungsgemäß einsetzbare Alkanolamine werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Erfindungsgemäß ganz besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Besonders bevorzugte Mittel (A) enthalten als Alkanolamin mindestens Monoethanolamin.

Bevorzugt sind die Alkanolamine in einer Menge von 0,05 bis 20 Gew.-%, insbesondere von 0,5 bis 15 Gew.-%, jeweils bezogen auf das Gesamtgewicht des anwendungsbereiten Aufhellmittels, enthalten.

Es zeigte sich jedoch in umfangreichen Untersuchungen, dass gerade stark alkalische Aufhellzubereitungen besondere Herausforderungen an schaumbildende Tenside stellen. Insbesondere schäumende Aufhellmittel mit hohen Mengen an Fettsäureamidoalkylbetainen als schaumbildende Tenside neigen, insbesondere in Gegenwart von Alkanolaminen als Alkalisierungsmittel, zur Entwicklung von Amin-artigen Gerüchen, die von Anwender als störend und unangenehm wahrgenommen werden.

Durch die Wahl bestimmter Betain-artiger Tenside konnte diese unerwünschte Nebenwirkung vollständig unterdrückt werden.

Das erfindungsgemäße Verfahren, ist dadurch gekennzeichnet, dass das Mittel (A) zusätzlich mindestens ein zwitterionisches Tensid der Formel (I), 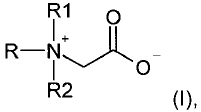 worin
- R: für eine gesättigte oder ungesättigte C₁₀-C₂₀-Alkylkette steht und
- R1 und R2: jeweils unabhängig voneinander für eine C₁-C₄-Alkylgruppe oder eine C₂-C₄-Hydroxyalkylgruppe stehen,
enthält.

Der Rest R steht dabei für eine Alkylkette mit 10 bis 20, vorzugsweise 10 bis 18 Kohlenstoffatomen, die eine oder mehrere Doppelbindungen ausweisen kann und gegebenenfalls verzweigt sein kann. Bevorzugte Beispiele solcher Alkylgruppen sind die Decyl-, Lauryl-, Myristyl-, Cetyl-, Palmoleyl-, 2-Hexyldecyl-, Stearyl-, Isostearyl-, Oleyl-, Elaidyl-, Petroselinyl-, Arachyl-, 2-Octyldodecyl oder Gadoleyl-Gruppe sowie deren Gemische, wie sie aufgrund von eingesetztem Rohstoff oder Herstellmethode anfallen. Bevorzugt sind für R Alkylgruppen auf Basis von Cocosalkyl- oder Talgfettalkylgruppen.

Die Reste R1 und R2 stehen unabhängig voneinander für eine C₁-C₄-Alkylgruppe oder eine C₂-C₄-Hydroxyalkylgruppe. Geeignete und bevorzugte C₁-C₄-Alkylgruppen sind dabei die Methyl-, Ethyl-, Propyl,- Isopropyl-, Butyl-, 1-Methylpropyl-, 2-Methylpropyl- und 1,1-Dimethylethyl-Gruppe. Geeignete und bevorzugte C₂-C₄-Hydroxyalkylgruppen sind dabei die 2-Hydroxyethyl-, 3-Hydroxypropyl- und 2-Hydroxypropyl-Gruppe. Besonders bevorzugt stehen R1 und R2 jeweils für eine Methylgruppe.

Eine Ausführungsform des ersten Erfindungsgegenstands ist dadurch gekennzeichnet, dass das Mittel (A) als zwitterionisches Tensid mindestens eine Verbindung der Formel (I) enthält, worin R1 und R2 jeweils für eine Methylgruppe und R für eine Cocosalkylgruppe stehen.

Solche besonders geeigneten zwitterionischen Tenside tragen die INCI-Bezeichnung Coco Betaine und werden beispielsweise als wässrige Lösung mit 30 Gew.-% Aktivsubstanz unter dem Handelsnamen Genagen® KB vertrieben.

Um einen stabilen Schaum zu erzeugen, ist es notwendig, dass das anwendungsbereite Aufhellmittel eine ausreichende Menge an zwitterionischem Tensid enthält. Eine Ausführungsform des ersten Erfindungsgegenstands ist daher dadurch gekennzeichnet, dass das Mittel (A) ein oder mehrere zwitterionische Tenside der Formel (I) in einem Gesamtgewichtsanteil von mindestens 2,5 Gew.-%, bevorzugt von mindestens 3 Gew.-% und insbesondere bevorzugt von mindestens 4 Gew.-%, bezogen auf das Gesamtgewicht des anwendungsbereiten Aufhellmittels, enthält.

Um die Schaumbildung und Schaumstabilität weiter zu verbessern, kann es erfindungsgemäß bevorzugt sein, weitere, insbesondere nichtionische Tenside dem Aufhellmittel zuzusetzen.

Eine weitere Ausführungsform des ersten Erfindungsgegenstands ist daher dadurch gekennzeichnet, dass das Mittel (A) zusätzlich mindestens ein nichtionisches Tensid, ausgewählt aus Alkylpolyglucosiden und/oder Alkenylpolyglucosiden und/oder ethoxylierten, nichtionischen Tensiden mit mindestens 30 Ethylenoxideinheiten, enthält.

Als bevorzugte nichtionische Tenside haben sich Alkylpolyglykoside sowie Alkylenoxid-Anlagerungsprodukte an gesättigte lineare Fettalkohole, Fettsäureester und Fettsäuren mit jeweils 30 bis 80 Mol Ethylenoxid pro Mol Fettalkohol bzw. Fettsäure erwiesen. Anwendungsbereite Zubereitungen mit hervorragenden Eigenschaften werden ebenfalls erhalten, wenn sie als nichtionische Tenside Fettsäureester von ethoxyliertem Glycerin enthalten.

Gemäß einer bevorzugten Ausführungsform enthält das Mittel (A) als nichtionisches Tensid mindestens ein Alkyl- und/oder Alkenylpolyglucosid. Alk(en)ylpolyglucoside stellen bekannte nichtionische Tenside gemäß Formel (II) dar,

R¹O-[G]ₚ (II)

in der R¹ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und Alkenylpolyglucoside können sich von Aldosen beziehungsweise Ketosen mit 5 oder 6 Kohlenstoffatomen, vorzugsweise von Glucose, ableiten. Die bevorzugten Alkyl- und/oder Alkenylpolyglucoside sind somit Alkyl- und/oder Alkenylpolyglucoside. Die Indexzahl p in der allgemeinen Formel (II) gibt den Polymerisierungsgrad (DP), d. h. die Verteilung von Mono- und Polyglucosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkylpolyglucosid eine analytisch ermittelte rechnerische Größe, die meistens eine gebrochene Zahl ist. Vorzugsweise werden Alkyl- und/oder Alkenylpolyglucoside mit einem mittleren Oligomerisierungsgrad p von 1,1 bis 3,0 eingesetzt. Aus anwendungstechnischer Sicht sind solche Alkyl- und/oder Alkenylpolyglucoside bevorzugt, deren Polymerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkylpolyglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkylpolyglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkylpolyglucoside auf Basis von gehärtetem C_{12/14}-Kokosalkohol mit einem DP von 1 bis 3.

Erfindungsgemäß geeignete Alkyl- und/oder Alkenylpolyglucoside werden unter der INCI-Bezeichnung Coco-Glucoside und dem Handelsnamen Plantacare 818 UP oder unter der INCI-Bezeichnung Lauryl-Glucoside und dem Handelsnamen Plantacare 1200 UP vertrieben.

Das beziehungsweise die Alkyl- und/oder Alkenylpolyglucoside sind in den erfindungsgemäßen Mitteln (A) bevorzugt in Mengen von 0,1 bis 20 Gew.-%, bezogen auf das anwendungsbereite Aufhellmittel, enthalten. Mengen von 1 bis 15 Gew.-% sind besonders bevorzugt. Mengen von 3 bis 8 Gew.-% sind ganz besonders bevorzugt.

Weiterhin hat es sich als vorteilhaft erwiesen, wenn die erfindungsgemäßen Mittel (A) anstelle oder gemeinsam mit den Alk(en)ylpolyglucosiden mindestens ein nichtionisches Tensid enthalten, ethoxylierten, nichtionischen Tensiden mit mindestens 30 Ethylenoxideinheiten.

Eine weitere Ausführungsform des ersten Erfindungsgegenstands ist daher dadurch gekennzeichnet, dass das Mittel als nichtionisches Tensid mindestens ein ethoxyliertes Tensid mit mindestens 30 Ethylenoxideinheiten enthält.

Neben den entsprechend ethoxylierten Fettalkoholen sind erfindungsgemäß insbesondere die Anlagerungsprodukte von 30 bis 60 Mol Ethylenoxid an Rizinusöl und gehärtetes Rizinusöl besonders geeignet. Diese speziellen nichtionischen Tenside ermöglichen eine weitere Verbesserung der Schaumkonsistenz, insbesondere im Hinblick auf eine höhere Festigkeit, eine gesteigerte Feinporigkeit und eine größere Geschmeidigkeit.

Beispiele für solche geeigneten Tenside tragen die INCI-Bezeichnungen Steareth-30. Ceteareth-30, Oleth-30, Ceteareth-50 oder PEG-40 Hydrogenated Castor Oil und PEG-60 Hydrogenated Castor Oil. PEG-60 Hydrogenated Castor Oil wird beispielsweise unter dem Handelsnamen Cremophor CO 60 vertrieben.

Das beziehungsweise die ethoxylierten, nichtionischen Tenside sind in den erfindungsgemäßen Mitteln (A) bevorzugt in Mengen von 0,1 bis 10 Gew.-%, bevorzugt von 0,5 bis 8 Gew.-% und besonders bevorzugt von 1,0 bis 5,0 Gew.-%, jeweils bezogen auf das anwendungsbereite Aufhellmittel, enthalten.

Das Mittel (B) gemäß dem erfindungsgemäßen Verfahren des ersten Erfindungsgegenstands enthält mindestens ein Oxidationsmittel. Als Oxidationsmittel kommen Persalze, Percarbonsäuren, Chlorite und insbesondere Wasserstoffperoxid oder dessen Anlagerungsprodukte an Harnstoff, Melamin sowie Natriumborat in Frage.

In einer bevorzugten Ausführungsform des ersten Erfindungsgegenstands ist das Oxidationsmittel des Mittels (B) Wasserstoffperoxid.

Das Oxidationsmittel ist in dem erfindungsgemäßen Mittel (B) bevorzugt in einem Anteil von 0,1 bis 10 Gew.-%, bevorzugt von 0,5 bis 8 Gew.-% und besonders bevorzugt von 1,0 bis 5,0 Gew.-%, jeweils bezogen auf das anwendungsbereite Aufhellmittel, enthalten.

Erfindungsgemäß bevorzugt ist auch der Einsatz von so genannten Komplexbildnern im Mittel (B). Komplexbildner sind Stoffe, die Metallionen komplexieren können. Dadurch wird die metallkatalysierte Zersetzung von Wasserstoffperoxid reduziert und so die Lagerstabilität der Mittel (B) erhöht. Bevorzugte Komplexbildner sind sogenannte Chelatkomplexbildner. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Ethylendiamindibernsteinsäure (EDDS), Nitrilotriessigsäure (NTA) und Hydroxyethandiphosphonsäuren bzw. deren Alkalisalze. Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz. Auch Dipicolinsäure wird erfindungsgemäß vorzugsweise als Komplexbildner eingesetzt. Mittel, die eine Kombination aus einem EDTA-Salz und HEDP und Dipicolinsäure enthalten sind erfindungsgemäß besonders bevorzugt.

Komplexbildner sind in dem erfindungsgemäßen Mittel (B) bevorzugt in einem Anteil von 0,005 bis 1,5 Gew.-%, bevorzugt von 0,01 bis 1 Gew.-% und besonders bevorzugt von 0,05 bis 0,5 Gew.-%, jeweils bezogen auf das anwendungsbereite Aufhellmittel, enthalten.

Das Mittel (C) des erfindungsgemäßen Verfahrens enthält mindestens ein Peroxosalz.
Bevorzugte Peroxosalze sind Peroxodisulfatsalze, insbesondere Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Aufhellmittels, enthalten sein.

Es hat sich herausgestellt, dass sich das Peroxosalz für eine gute Anmischbarkeit und daher für eine gleichmäßige, einheitliche Auftragung durch eine schnelle Löslichkeit während des Vermischens der Mittel (A), (B) und (C) auszeichnen sollte. Dies ermöglicht darüber hinaus eine verbesserte Ausbringbarkeit des Mittels aus dem Schaumspendergefäß im zweiten Verfahrensschritt ii des erfindungsgemäßen Verfahrens.

Es zeigte sich, dass insbesondere Natriumpersulfat (= Natriumperoxodisulfat) und Ammoniumpersulfat (Ammoniumperoxodisulfat) sich hierzu besonders eignet.

In einer Ausführungsform des ersten Erfindungsgegenstands ist daher dadurch gekennzeichnet, dass das Mittel (C) als Peroxosalz Natriumpersulfat und/oder Ammoniumpersulfat enthält.

Das Mittel (C) kann weitere Komponenten enthalten. Insbesondere bei Konfektionierung in Pulverform kann es bevorzugt sein, dem Mittel (C) zusätzliche Verdicker, wie Cellulose, Cellulosederivate, Xanthan oder Acrylsäurepolymere, Entstaubungsmittel, wie Mineralöle, oder weitere Blondieraktivatoren, wie Carbonate oder Kieselsäure (Silica) zuzugeben.

Die Vermischung der Mittel (A), (B) und (C) im ersten Schritt des erfindungsgemäßen Verfahrens erfolgt durch Zusammenführen der Mittel in einem geeigneten Gefäß. Hierbei hat es sich als vorteilhaft erwiesen, die Vermischung direkt im Schaumspendergefäß des zweiten Verfahrensschritts durchzuführen.

Das anwendungsbereite Aufhellmittel kann je nach Bedürfnis des Anwenders weitere Inhaltsstoffe enthalten, die entweder in einem oder in mehreren der Mittel (A), (B) und (C) enthalten waren.

Es konnte in den dieser Erfindung zugrunde liegenden Arbeiten gezeigt werden, dass es vorteilhaft ist, wenn die anwendungsbereite Aufhellmittel neben den oben genannten Tensiden mindestens ein anionisches Tensid enthalten. Als anionische Tenside sind
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist, und
- Alkylsulfate und Alkylpolyglycolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
erfindungsgemäß besonders bevorzugt.

In einer ganz besonders bevorzugten Ausführungsform der vorliegenden Erfindung enthalten die anwendungsbereiten Aufhellmittel neben den erfindungswesentlichen Tensiden weiterhin ein anionisches Tensid. Es sich als vorteilhaft erwiesen, wenn das anionische Tensid in der Oxidationsmittelzubereitung (Mittel (B)) und die übrigen Tenside im Mittel (A) enthalten sind.

Weiterhin hat es sich als erfindungsgemäß als wesentlich zur Erzielung der gewünschten Schaumeigenschaften erwiesen, wenn das anwendungsbereite Aufhellmittel nach Vermischen aus den drei Mittel (A), (B) und (C) einen Gesamttensidgehalt von mindestens 10 Gew.-% aufweist. Mittel, die mindestens 11 Gew.-%, vorzugsweise mindestens 12 Gew.-% Gesamttensidgehalt, jeweils bezogen auf das anwendungsbereite Aufhellmittel, aufweisen sind insbesondere bevorzugt.

Außerdem hat es sich als vorteilhaft erwiesen, wenn die Anwendungszubereitung frei von kationischen Tensiden formuliert ist.

Zur Nuancierung der resultierenden Aufhelltöne kann es erfindungsgemäß bevorzugt sein, wenn das anwendungsbereite Aufhellmittel weiterhin mindestens einen direktziehenden Farbstoff enthält. Hierbei handelt es sich um Farbstoffmoleküle, die direkt auf das Substrat aufziehen und keinen oxidativen Prozess zur Ausbildung der Farbe benötigen. Die direktziehenden Farbstoffe werden jeweils bevorzugt in einer Menge von 0,001 bis 20 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 sowie Tetrabromphenolblau und Bromphenolblau bekannten Verbindungen.

Bevorzugte kationische direktziehende Farbstoffe sind dabei kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist. Die Verbindungen, die auch unter den Bezeichnungen Basic Yellow 87, Basic Orange 31 und Basic Red 51 bekannt sind, sind ganz besonders bevorzugte kationische direktziehende Farbstoffe.

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Weiterhin ist es möglich, neben der Aufhellung eine oxidative Färbung der keratinischen Fasern zu erreichen, wenn das anwendungsbereite Aufhellmittel zusätzlich mindestens ein Oxidationsfarbstoffvorprodukt enthält. Zur Lagerstabilität sind in diesem Fall die Oxidationsfarbstoffvorprodukte bevorzugt im Mittel (A) konfektioniert. Oxidationsfarbstoffvorprodukte bilden ihre Färbung aus mindestens einer Entwicklerkomponente sowie gegebenenfalls mindestens einer Kupplerkomponente aus.

Bevorzugte Entwicklerkomponenten werden ausgewählt aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Aufhellmittel, verwendet.

Erfindungsgemäß bevorzugte Kupplerkomponenten werden ausgewählt aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Aufhellmittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Überraschenderweise konnte gezeigt werden, dass die Anwesenheit geringer Mengen eines polymeren Verdickers die Schaumbeständigkeit positiv beeinflussen konnte. Daher ist es im Rahmen einer Ausführungsform der vorliegenden Erfindung bevorzugt, wenn die anwendungsbereiten Aufhellmittel mindestens einen polymeren Verdicker enthalten.

Beispiele für erfindungsgemäß bevorzugte polymere Verdicker sind: Acrylates Copolymer, Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, AcrylatesNinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/lsophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene /Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 ,Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates /Vinyl Isodecanoate Crosspolymer, Sodium AcrylateNinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/ Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136 / HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Aus dieser umfangreichen Gruppe haben sich die Verdickungsmittel als besonders vorteilhaft erwiesen, die mindestens ein Monomer vom Typ der Acrylsäure oder Methacrylsäure sowie derer Derivate enthalten. Ein erfindungsgemäß ganz besonders bevorzugtes Polymer ist das unter der INCI-Bezeichnung Acrylates Copolymer bekannte Copolymer aus zwei oder mehr Monomeren, ausgewählt aus Acrylsäure, Methacrylsäure und deren Ester mit C₁-C₄-Alkylgruppen.

Es ist besonders vorteilhaft, wenn der polymere Verdicker in geringen Mengen, vorzugsweise in Mengen von 0,05 bis 2,5 Gew.-%, insbesondere von 0,3 bis 1,5 Gew.-%, jeweils bezogen auf das anwendungsbereite Aufhellmittel, enthalten ist.

Eine weitere Verbesserung der pflegenden Eigenschaften des Produktes konnte durch den Einsatz eines Extraktes, der aus und/oder mithilfe von Algen und/oder Plankton gewonnen wird, erzielt werden. Insbesondere der Feuchtigkeitshaushalt der Fasern als auch deren Glanz konnte durch diese Extrakte erheblich gesteigert werden. Unter "Extrakten, die aus und/oder mithilfe von Algen und/oder Plankton gewonnen werden" sind erfindungsgemäß Wirkstoffmischungen zu verstehen, die entweder durch Extraktion von Algen und/oder Plankton selbst oder durch Extraktion der die Algen und/oder das Plankton umgebenen Wasserphase gewonnen werden. Erfindungsgemäß bevorzugte Algen und/oder Planktonarten sind ausgewählt aus den Gattungen Haptophyta, Schlundgeißler (Cryptista), Euglenozoa, Dinozoa, Chlorarachniophyta, Goldalgen (Chrysophyta), Kieselalgen (Bacillariophyta, auch Diatomeen genannt), Braunalgen (Phaeophyta), Dinogellaten, Rotalgen (Rhodophyta), Grünalgen (Chlorophyta), Picobiliphyta sowie der Blaualgen (beispielsweise Oscillatoria und Spirulina). Besonders bevorzugt sind im Rahmen der vorliegenden Erfindung Extrakte aus vornehmlich in Süßwasser vorkommenden Blaualgen.

Hinsichtlich der Art und Weise, wie die erfindungsgemäßen Extrakte aus den Algen- und/oder Planktonbestandteilen gewonnen wird, bestehen prinzipiell keine Einschränkungen. Als Extraktionsmittel zur Herstellung der genannten Algenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Blaualgenextrakte, die mittels einer Wasser/Propylenglykol-Mischung gewonnen werden, haben sich als besonders geeignet erwiesen. Es hat sich dabei als besonders geeignet erwiesen, wenn diese Extraktionsmittel in einem Verhältnis von 1:10 bis 10:1 eingesetzt werden.

Weiterhin kann es erfindungsgemäß bevorzugt sein, Extrakte einzusetzen, die vor der Anwendung zumindest teilweise entfärbt wurden. Dies kann beispielsweise durch Nutzung von Aktivkohle erfolgen. Ebenso ist möglich, in den erfindungsgemäßen Mitteln als Algen- oder Plankton-Extrakt die wässrige Aufzucht- beziehungsweise Kulturlösung von Algen oder Plankton einzusetzen. Dazu werden die Algen oder das Plankton zunächst mit einer physikalischen Trennmethode, wie Filtration oder Zentrifugation von der Kulturlösung abgetrennt.

In einer besonders bevorzugten Ausführungsform der vorliegenden Erfindung enthält das anwendungsbereite Aufhellmittel einen Extrakt einer Blaualge, vorzugsweise einer Süßwasserblaualge, ganz besonders bevorzugt einer Blaualge der Gattung Spirulina.

Die erfindungsgemäßen anwendungsbereite Aufhellmittel enthalten die Algen- und/oder Planktonextrakte vorzugsweise in einer Menge von 0,001 bis 5 Gew.-%, vorzugsweise von 0,01 bis 2 Gew.-% bezogen auf das anwendungsbereite Mittel.

Weiterhin kann es erfindungsgemäß bevorzugt sein, wenn die Mittel zusätzlich mindestens eine Aminosäure und/oder mindestens ein Protein. Erfindungsgemäß bevorzugte Aminosäuren sind Arginin, Serin, Lysin, Glycin, Tyrosin, Prolin, Glutamin, Cystein und Histidin. Der Aminosäuren und/oder die Proteine ermöglicht eine überraschend stark ausfallende Strukturgebung der Haare.

Ferner konnten die Erfinder der vorliegenden Anmeldung zeigen, dass es sich vorteilhaft auf die Beschaffenheit des Schaums auswirkt, wenn die anwendungsbereiten Aufhellmittel frei von Silikonölen formuliert sind.

Ferner können die erfindungsgemäßen anwendungsbereite Aufhellmittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/VinylacetatCopolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikoripolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide , insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; pflanzliche Öle; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, B₃, B₅, B₆, C, E, F und H; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente.

Die Auswahl dieser weiteren Stoffe wird der Fachmann gemäß den gewünschten Eigenschaften der anwendungsbereiten Aufhellmittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, jeweils bezogen auf das Gesamtgewicht der anwendungsbereite Aufhellmittel, eingesetzt.

Das anwendungsbereite Aufhellmittel besitzt bevorzugt einen pH-Wert im Bereich von 6 bis 12 aufweisen. Besonders bevorzugt ist die Anwendung der Aufhellmittel in einem alkalischen Milieu. Bei den pH-Werten im Sinne der vorliegenden Erfindung handelt es sich um pH-Werte, die bei einer Temperatur von 22 °C gemessen wurden.

Weiterhin hat es sich als besonders bevorzugt erwiesen, wenn das resultierende, anwendungsbereite Aufhellmittel dünnflüssig formuliert ist. Anwendungsbereite Aufhellmittel, die nach Vermischung im ersten Verfahrensschritt eine Viskosität von 0 bis 2000 mPas aufweisen (gemessen bei 22°C im Brookfield-Viskosimeter Typ RV-T mit Spindel LV-1 beziehungsweise RV-1 und einer Geschwindigkeit von 30U/min), haben sich als besonders bevorzugt erwiesen. Eine Viskosität von 0 bis 1000mPas, gemessen unter den genannten Bedingungen, ist erfindungsgemäß besonders bevorzugt. Eine Viskosität von 5 bis 500 mPas, insbesondere von 10 bis 50 mPas (gemessen untern den oben genannten Bedingungen) ist erfindungsgemäß ganz besonders bevorzugt.

Im zweiten, erfindungsgemäßen Verfahrensschritt wird das anwendungsbereite Aufhellmittel aus einem Schaumspendergefäß ausgebracht und auf die aufzuhellenden Fasern aufgebracht. Es hat sich als vorteilhaft erwiesen, die Vermischung der Mittel (A), (B) und (C) im ersten Schritt des erfindungsgemäßen Verfahrens direkt im Schaumspendergefäß des zweiten Verfahrensschritts durchzuführen.

Es ist erfindungsgemäß bevorzugt, wenn das anwendungsbereite Aufhellmittel in einem geeigneten Spender aufgenommen und zur jeweiligen Verwendung abgegeben wird. Dabei erfolgt die Ausgabe der anwendungsbereiten Aufhellmittel grundsätzlich in Schaumform. Die schaumförmige Konsistenz der Zubereitung ist in diesem Zusammenhang sehr breit zu verstehen und umfasst jedwede Mischung von einer fließfähigen Zubereitung und einer gasförmigen Komponente. Insofern sind sowohl fließfähige als auch im Wesentlichen feste, stabile Schaumkonsistenzen vom Erfindungsgegenstand umfasst.

Erfindungsgemäß ist ein Schaum als Gas-Flüssigkeits-Gemisch definiert. Ein Schaum kennzeichnet dabei Gebilde aus gasgefüllten, kugel- oder polyederförmigen Zellen (Poren), welche durch flüssige, halbflüssige oder hochviskose Zellstege begrenzt werden. Erfindungsgemäß bevorzugt enthält der Schaum als Gas Luft und/oder Gemische von Luft und Reaktionsgasen, die beim Vermischen der Mittel (A), (B) und (C) entstehen.

Der Gasanteil des Schaums beträgt vorzugsweise mindestens 50 Vol-%, bevorzugt mindestens 70 Vol-% und weiter bevorzugt mindestens 80 Vol-%, jeweils bezogen auf das Gesamtvolumen des anwendungsbereiten Mittels.

Wenn die Volumenkonzentration des den Schaum bildenden Gases bei homodisperser Verteilung kleiner als 74% ist, so sind die Gasblasen wegen der oberflächenverkleinernden Wirkung der Grenzflächenspannung kugelförmig. Oberhalb der Grenze der dichtesten Kugelpackung werden die Blasen zu polyedrischen Lamellen deformiert, die von ca. 4 bis 600 nm dünnen Häutchen begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum.

Erfindungsgemäß besonders geeignete Schäume weisen ein Gas-Flüssigkeits-Verhältnis von 5 bis 50 mL/g, bevorzugt 10 bis 40 mL/g und insbesondere 15 bis 35 mL/g auf. Ein solches Gas-Flüssigkeits-Verhältnis wird dabei beispielsweise dadurch bestimmt, dass nach abgeschlossenem Mischvorgang bei Raumtemperatur und einer Ruhezeit von 1 min das Volumen des Schaums, beispielsweise mittels Messzylinder, bei bekanntem Gewicht der Mittel (A), (B) und (C) bestimmt wird. Alternativ kann auch ein bestimmtes Volumen, beispielsweise durch Abgabe in einen Messzylinder entnommen werden und dessen Gewicht durch Auswaage bestimmt werden.

Grundsätzlich umfasst ein erfindungsgemäßes Schaumspendergefäß, kurz Spender zumindest einen Vorratsbehälter zur Aufnahme wenigstens einer Komponente der Aufhellmittel und eine Applikationsvorrichtung zur Ausgabe der Aufhellmittel in Form von Schaum. Dabei ist der Vorratsbehälter insbesondere als tuben- oder fläschenförmiger Behälter gestaltet, während die Applikationsvorrichtung diesen einseitig offenen Behälter verschließt. Die eigentliche Zubereitungsabgabe wird bevorzugt mittels einer geeigneten Druckquelle bewirkt, welche in den Spender, insbesondere den Vorratsbehälter, integriert ist, oder aber mittels manuellem Druckaufbau durch den Benutzer des Aufhellmittels selbst veranlasst.

Als Beispiel für erfindungsgemäße Spender mit integrierter Druckquelle sind Druckbehälter zu nennen, die üblicherweise im Behälterinneren entweder einen geeigneten Druckspeicher aufweisen, z. B. einen mechanischen, oder aber ein Treibmittel enthalten und auf diesem Wege das Innere des Behälters unter Druck setzen. Derartige Druckbehälter verfügen üblicherweise über geeignete Ventilvorrichtungen zur Ausgabe der im Inneren des Druckbehälters befindlichen Zubereitung während einer entsprechenden Ventilbetätigung. Solche Druckbehälter sind vor allem in Verbindung mit gasförmigen und/oder flüssigen Treibmitteln in Form von Aerosolspendern für unterschiedlichste kosmetische Anwendungen vorbekannt, z. B. Haarstylingspray, Haarfärbezubereitungen, Deospray, Rasierschaum/-gel etc..

Alternativ können erfindungsgemäß auch manuell betätigte Spender zum Einsatz kommen, die sich lediglich der Krafteinwirkung des Nutzers bedienen, um eine schaumförmige Zubereitungsabgabe zu bewirken. Bei diesen Bauformen kann vorteilhaft auf eine zusätzliche Druckquelle, z. B. Treibmittel, verzichtet werden, was vor allem aus Kosten- sowie Nachhaltigkeitsgründen wünschenswert ist. Solche durch manuelle Krafteinwirkung betätigbaren Schaumspender sorgen neben der Förderung des Aufhellmittels aus dem Vorratsbehälter zur Abgabeöffnung hin parallel auch für eine entsprechende Aufschäumung der Aufhellmittel. Während dieser Aufschäumung oder Schaumbildung wird grundsätzlich das Aufhellmittel mit einer gasförmigen Komponente, insbesondere Luft, vermischt. Im Einzelnen ist dazu eine Schäumvorrichtung vorgesehen, die dies bewerkstelligt.

Gemäß einer ersten Variante eines manuell betätigbaren Spenders ist dieser als Schüttelspender ausgeführt, mit mindestens einem Vorratsbehälter zur Aufnahme des Aufhellmittels und einer zugehörigen Abgabevorrichtung zur schaumförmigen Abgabe des Aufhellmittels. Dabei ist die Abgabevorrichtung, insbesondere lösbar, mit dem Vorratsbehälter verbunden. Die eigentliche Schaumbildung erfolgt innerhalb des Schüttelspenders durch Agitation des Aufhellmittels innerhalb des Vorratsbehälters. In sofern bildet der Schüttelspender in Verbindung der entsprechenden Spenderbewegung die oben genannte Schäumvorrichtung. Im Anschluss an diese Art der Aufschäumung kann dann die Abgabe des schaumförmigen Aufhellmittels mittels der Abgabevorrichtung erfolgen.

Eine weitere sinnvolle Spendervariante ergibt sich durch Ausgestaltung als Quetsch- oder Squeeze-Schaumspender. Ein solcher Quetsch-Schaumspender besitzt neben dem mindestens einen Vorratsbehälter zur Aufnahme des Aufhellmittels eine entsprechende Applikationsvorrichtung, innerhalb der die Aufschäumung sowie anschließende Abgabe des Aufhellmittels erfolgt. Die eigentliche Förderung des Aufhellmittels aus dem Vorratsbehälter wird mittels Krafteinwirkung auf die flexible Vorratsbehälterwandung bewirkt. Dabei sorgt die reversible Verformung der Vorratsbehälterwandung für einen Druckaufbau im Inneren des Vorratsbehälters, so dass als Folge das Aufhellmittel aus dem Vorratsbehälter getrieben wird. Dazu ist es notwendig die Vorratsbehälterwandung hinreichend flexibel bzw. reversibel verformbar zu gestalten. Dies wird gewährleistet durch eine anwendungsbezogen zielgerichte Auslegung der Dicke der Vorratsbehälterwandung in Verbindung mit einer geeigneten Materialauswahl für die Vorratsbehälterwandung. Bevorzugt wird die Vorratsbehälterwandung eines entsprechenden Quetsch-Schaumspenders aus einem Polyolefin, wie beispielsweise Polypropylen (PP), high density Polyethylen (HDPE), medium density Polyethylen (MDPE), low density Polyethylen (LDPE), linear low density Polyethylen (LLDPE), ausgeführt. Darunter ist Polypropylen (PP) bevorzugt geeignet.

Die Applikationsvorrichtung eines solchen Quetsch-Schaumspenders umfasst zur Aufschäumung des Aufhellmittels auch eine entsprechende Schäumvorrichtung. Die Schäumvorrichtung ist in der Lage eine Zubereitungsmenge mit einer Gasmenge im geeigneten Dosierverhältnis zu vermischen, um die gewünschte Schaumkonsistenz des Aufhellmittels auszubilden. Üblicherweise wird hierzu ein eingezogener Zubereitungsstrom mit einem eingezogenen Gasstrom innerhalb einer Mischkammer der Schäumvorrichtung zusammen geführt und dort durch strömungstechnische Verwirbelung vermengt. Besonders bevorzugt wird als gasförmige Komponente zur Schaumbildung Luft verwendet, die entweder unmittelbar aus der Vorratsbehälter oder der Umgebung eingezogen wird.

Die grundsätzliche Funktionsweise derartiger Quetsch-Schaumspender wird auch in den Dokumenten WO 2007/086730 A2/A3 sowie EP 1237660 B1 beschrieben. Ein erfindungsgemäßer Quetsch-Schaumspender kann auch entsprechend zu diesen Patentdokumenten ausgebildet sein. Insbesondere kann der erfindungsgemäße Quetsch-Schaumspender gemäß der Offenbarung von EP 1237660 B1 so ausgeführt werden, dass eine Verwendung des Quetsch-Schaumspenders in im Wesentlichen aufrechter Position als auch in Überkopfstellung möglich ist.

Analog kann der Spender auch als Pump-Schaumspender ausgebildet sein mit mindestens einer Vorratsbehälter zur Aufnahme des Aufhellmittels sowie einer Applikationsvorrichtung, wobei in diesem Fall die Applikationsvorrichtung eine Pumpvorrichtung zur Förderung sowohl des Aufhellmittels als der gasförmigen Komponente, bevorzugt Luft, aufweist und darüber hinaus eine entsprechende Schäumvorrichtung umfasst. Im Detail ergibt sich die Funktionsweise und der konstruktive Aufbau derartiger Pump-Schaumspender u. a. auch aus den Patentdokumenten WO 2007/083206 A1 oder WO 2007/091882 A1. Insbesondere kann der erfindungsgemäße Pump-Schaumspender gemäß der Offenbarung dieser genannten Dokumente ausgebildet sein.

Bei Verwendung der genannten Spendervarianten in Verbindung mit mehrkomponentigen Mitteln, muss sichergestellt werden, dass die Einzelkomponenten bis zur eigentlichen Zubereitungsanwendung getrennt bevorratet werden. Das Aufhellmittel wird erfindungsgemäß mit einer der oben beschriebenen Spendervarianten mit nur einem Vorratsbehälter und einer Applikationsvorrichtung angewendet werden. Dafür ist der Vorratsbehälter derart gestaltet, dass er sich wieder verschließbar öffnen lässt. Idealerweise ist der Vorratsbehälter mittels der Applikationsvorrichtung verschlossen, wobei die Applikationsvorrichtung lösbar mit dem Vorratsbehälter verbunden ist, beispielsweise über eine Schraub- oder Rastverbindung. Dies eröffnet die Möglichkeit den Vorratsbehälter bereits bei der Herstellung mit einer Komponente des Aufhellmittels vorzukonfektionieren und weitere Komponenten des Aufhellmittels erst kurz vor der eigentlichen Anwendung des Aufhellmittels in den Vorratsbehälter zuzugeben. Hierbei sind die weiteren Komponenten dem gesamten Aufhellmittel in Form eines Kits innerhalb von geeigneten separaten Behältern beigegeben und werden vom Nutzer unmittelbar vor der Anwendung des Aufhellmittels im Vorratsbehälter gemischt.

Für alle aufgeführten Spendervarianten kann innerhalb der jeweiligen Applikationsvorrichtung die Durchmischung der Einzelkomponenten des Aufhellmittels erleichtert bzw. verbessert werden, durch Verwendung geeigneter zusätzlicher Mischvorrichtungen, z. B. statischer Mischer (wie auch in WO 2005/102539 A1 beschrieben) oder poröser Einsatzelemente. Derartige Mischvorrichtungen lassen sich vorteilhaft an geeigneter Stelle innerhalb eines Strömungskanals für das Aufhellmittel in der Applikationsvorrichtung anordnen.

Darüber hinaus können zusätzlich ein oder mehrere poröse Einsatzelemente eingesetzt werden, um die innerhalb der Schäumvorrichtung erzielbare Schaumkonsistenz positiv zu beeinflussen. Solche porösen Einsatzelemente sind beispielsweise schwamm- oder netzartig gestaltet und sind innerhalb der Schäumvorrichtung an geeigneter Stelle im Strömungskanal für die Aufhellmittel positioniert, etwa unmittelbar stromaufwärts der Spenderabgabeöffnung. Das poröse Einsatzelement gestattet somit die Durchströmung des Aufhellmittels und sorgt infolge strömungstechnischer Verwirbelung für eine feinere und homogenere Schaumkonsistenz. In Abhängigkeit von der jeweiligen Gestaltung des porösen Einsatzelementes lässt sich somit die Schaumkonsistenz direkt beeinflussen. Bei Verwendung eines netzartigen Einsatzelementes erweist es sich als sinnvoll das netzartige Einsatzelement bevorzugt mit einer Lochdichte von 50 bis 220 mesh (mesh = Anzahl der Löcher pro Inch), besonders bevorzugt von 90 bis 200 mesh, ganz besonders bevorzugt von 125 bis 175 mesh auszubilden. Bei Verwendung mehrerer netzartiger Einsatzelemente können auch Einsatzelemente mit unterschiedlichen Lochdichten zum Einsatz gelangen. Hierbei weist das erste, stromaufwärts positionierte netzartige Einsatzelement bevorzugt eine Lochdichte von 50 bis 220 mesh (mesh = Anzahl der Löcher pro Inch), besonders bevorzugt von 90 bis 200 mesh, ganz besonders bevorzugt von 125 bis 175 mesh auf. Das zweite, stromabwärts positionierte Netz weist bevorzugt eine Lochdichte von 160 bis 280 mesh, besonders bevorzugt von 175 bis 245 mesh und ganz besonders bevorzugt von 180 bis 220 mesh, auf. Letztlich wird die Anzahl der verwendeten porösen Einsatzelemente wie auch deren spezifische Lochdichte bzw. deren Porösitätseigenschaften in Abhängigkeit vom jeweiligen Anwendungsfall zielgerichtet ausgelegt.

Die Verteilung des anwendungsbereiten Aufhellmittels auf den Fasern, insbesondere menschlichen Kopfhaar, erfolgt durch den Anwender vorzugsweise mit den Händen, gegebenenfalls auch mit einem technischen Hilfsmittel, wie Kamm, Bürste oder Pinsel. Dabei kann das anwendungsbereite Aufhellmittel als ausgebrachter Schaum unmittelbar auf den Haaransatz aufgetragen und anschließend mit den Händen oder einem mechanischen Hilfsmittel auf den Fasern verteilt werden. Es ist aber auch denkbar, dass der Schaum zunächst auf ein mechanisches Hilfsmittel, wie einen Kamm, aufgebracht wird und anschließend mit dessen Hilfe auf den Fasern verteilt wird. Unabhängig von der Art der Auftragung kann es erfindungsgemäß bevorzugt sein, wenn der Schaum anschließend in die Haare einmassiert wird.

Im dritten Verfahrensschritt des erfindungsgemäßen Verfahrens des ersten Erfindungsgegenstands verbleibt das Aufhellmittel während einer Einwirkungszeit von 1 bis 60 min, vorzugsweise von 5 bis 45 min auf den aufzuhellenden Fasern.

Die Anwendungstemperaturen des Aufhellmittels können in einem Bereich zwischen 15 und 40 °C liegen. Gegebenenfalls kann auch eine externe Wärmequelle zur Wärmezufuhr eingesetzt werden. Besonders bevorzugt ist es, die Aufhellung durch physikalische Maßnahmen zu unterstützen. Erfindungsgemäße Verfahren, bei denen die Anwendung durch Einwirkung von Wärme, IR- und/oder UV-Strahlung während der Einwirkzeit unterstützt wird, sind bevorzugt.

Im vierten Verfahrensschritt wird schließlich das verbliebene Aufhellmittel aus den Fasern durch Ausspülen der aufzuhellenden Fasern entfernt.

Je nach Tensidgehalt des anwendungsbereiten Aufhellmittels kann das Ausspülen mit Wasser oder einem tensid-haltigen Mittel, wie beispielsweise einem Shampoo erfolgen.

Je nach Wunsch des Anwenders kann dem Verfahren noch weitere Schritte zugefügt werden, wie beispielsweise zusätzliche Faserpflegeschritte mit einem Konditioniermittel oder Verformungsschritte zur Gestaltung der Frisur im Falle einer Anwendung auf menschlichem Kopfhaar.

Ein zweiter Erfindungsgegenstand der vorliegenden Anmeldung ist ein Mittel zur Aufhellung keratinischer Fasern, welches durch Vermischen aus drei getrennt voneinander konfektionierten Zubereitungen hergestellt ist, und welches dadurch gekennzeichnet ist, dass eine der Zubereitungen ein Mittel (A), dass eine der Zubereitungen ein Mittel (B) sowie dass eine der Zubereitungen ein Mittel (C) gemäß dem ersten Erfindungsgegenstand darstellt, wobei dass das Mittel (A) zusätzlich mindestens ein zwitterionisches Tensid der Formel (I), 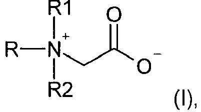 worin
- R: für eine gesättigte oder ungesättigte C₁₀-C₂₀-Alkylkette steht und
- R1 und R2: jeweils unabhängig voneinander für eine C₁-C₄-Alkylgruppe oder eine C₂-C₄-Hydroxyalkylgruppe stehen,
enthält.

Eine bevorzugte Ausführungsform des zweiten Erfindungsgegenstands ist daher dadurch gekennzeichnet, dass das anwendungsbereite Aufhellmittel in Form eines Schaums vorliegt.

Es kann bevorzugt sein, die Mittel (A), (B) und (C) getrennt voneinander zu lagern. Zweckmäßigerweise werden die Mittel dem Anwender jedoch in einer Umverpackung bereit gestellt.

Ein dritter Gegenstand der vorliegenden Erfindung ist ein Kit zur Aufhellung keratinischer Fasern, dadurch gekennzeichnet, dass es mindestens drei getrennt voneinander konfektionierte Komponenten aufweist, wobei die erste Komponente ein Mittel (A), enthaltend mindestens ein Alkalisierungsmittel, die zweite Komponente ein Mittel (B), enthaltend mindestens ein Oxidationsmittel, und die dritte Komponente ein Mittel (C), enthaltend mindestens ein Peroxosalz, enthält, wobei dass das Mittel (A) zusätzlich mindestens ein zwitterionisches Tensid der Formel (I), 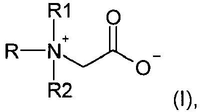 worin
- R: für eine gesättigte oder ungesättigte C₁₀-C₂₀-Alkylkette steht und
- R1 und R2: jeweils unabhängig voneinander für eine C₁-C₄-Alkylgruppe oder eine C₂-C₄-Hydroxyalkylgruppe stehen,
enthält.

Die voneinander getrennt konfektionierte Mittel oder Komponenten werden dazu in physikalisch getrennten Containern bereitgestellt. Der Begriff "Container" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff "Container" umfasst daher - ohne darauf beschränkt zu sein, das Innere einer Tube, eines Beutels, Tütchens oder Sackes, eines Kanisters, einer Dose, einer Wanne, einer Flasche, eines Glases oder eines Paketes, eines Kartons, einer Box, eines Umschlages oder eines anderen Behältnisses. Die Komponenten der Zubereitungen können in einem einzelnen Container, der mehrere Kompartimente zur Aufnahme der Zubereitungen aufweist, enthalten sein, es ist aber auch möglich und gegebenenfalls bevorzugt, sie auf verschiedene Container aufzuteilen.

Zur Vermischung und Abgabe als Schaum enthält die Mehrverpackungseinheit dabei zusätzlich mindestens ein Schaumspendergefäß, welches wie bereits erläutert, auch als Vorratsgefäß für eines der Mittel (A), (B) oder (C) eingesetzt werden kann.

Eine Ausführungsform des dritten Erfindungsgegenstands ist daher dadurch gekennzeichnet, dass die Mehrkomponentenverpackungseinheit weiterhin ein Spendergefäß enthält, das geeignet ist, die anwendungsbereite Mischung der Mittel (A), (B) und (C) in Form eines Schaums abzugeben.

Bezüglich weiterer bevorzugter Ausführungsformen des erfindungsgemäßen Kits gilt mutatis mutandis das zu den erfindungsgemäßen Verfahren und Mitteln Gesagte.

### Vergleichsbeispiele

Es wurden die folgenden Zubereitungen hergestellt. Die Mengenangaben verstehen sich, soweit nichts anderes vermerkt ist, jeweils in Gewichtsprozent.

### 1 Blondiercreme (Mittel A)

| Rohstoff | A |
|---|---|
| Plantacare 818 UP | 25,00 |
| Genagen KB | 30,00 |
| Cremophor CO 60 | 3,00 |
| Tetranatrium-EDTA | 0,20 |
| Natriumsulfit | 0,10 |
| Ascorbinsäure | 0,05 |
| L-Serin | 1,00 |
| Monoethanolamin | 8,00 |
| Eau Vitale d'algue bleue | 2,00 |
| Merquat 281 | 3,00 |
| Parfum | qs |
| Wasser | ad 100 |

### 2 Entwicklerzubereitungen (Mittel B)

| Rohstoff | B |
|---|---|
| Dipicolinsäure | 0,10 |
| Dinatriumpyrophosphat | 0,03 |
| Aculyn 33A | 2,50 |
| Texapon NSO | 2,00 |
| Natriumhydroxid (wässrig, 45%ig) | 0,73 |
| Turpinal SL | 1,50 |
| Wasserstoffperoxid (wässrig, 50 %ig) | 15,20 |
| Wasser | ad 100 |

### 3 Blondierpulver (Mittel C)

| Rohstoff | C1 | C2 |
|---|---|---|
| Natriumpersulfat | 100 | -- |
| Ammoniumpersulfat, 98% + Silica, 2% | -- | 100 |

### 4 Verzeichnis der eingesetzten Handelsprodukte

| | |
|---|---|
| Aculyn 33A | ca. 28% Festkörper in Wasser; INCI-Bezeichnung: Acrylates Copolymer |
| Cremophor CO60 | INCI-Bezeichnung: PEG-60 Hydrogenated Castor Oil (BASF) |
| Merquat 281 | ca. 40% Aktivsubstanz; INCI-Bezeichnung: Aqua (Water), Polyquaternium-22 (Cognis) |
| Eau Vitale d'algue bleue | ca. 0,1-0,99 Gew.-% Aktivsubstanz; INCI-Bezeichnung: Aqua (Water), Plankton Extract, Penoxyethanol (Soliance) |
| Plantacare 818UP | ca. 51-53% Aktivsubstanzgehalt; INCI-Bezeichnung: Coco-Glucoside, Aqua (Water) (Cognis) |
| Texapon NSO | ca. 27,5% Aktivsubstanz; INCI-Bezeichnung: Sodium Laureth Sulfate (Cognis) |
| Turpinal SL | ca. 58 - 61% Aktivsubstanzgehalt; INCI-Bezeichnung: Etidronic Acid, Aqua (Water) (Solutia) |

### 5 Aufhellung

### 5.1 Anwendung mit einem Quetsch-Schaumspender

35 g des Mittels B wurde in einem Quetsch-Schaumspender vorgelegt. Unmittelbar vor der Anwendung wurden 35 g des Mittel B und 10 g des Mittels (C1) oder (C2) vorsichtig zugegeben, so dass keine Schaumbildung erfolgte. Anschließend wurde die Squeezeflasche mehrfach vorsichtig auf den Kopf und wieder zurückgedreht, so dass eine gründliche Durchmischung der Komponente bei geringer Schaumbildung stattfand.

Der Schaum wurde durch Drücken des Quetsch-Schaumspenders direkt auf den Haaransatz eines Probanden ausgebracht, anschließend die Haarlängen benetzt und zum Schluss mit einem Kamm gleichmäßig in den Fasern verteilt. Nach einer Einwirkzeit von 45 min bei Raumtemperatur wurden die Haare gründlich mit Wasser gespült, shampooniert und mit einem Haartrockner getrocknet.

### 5.2 Anwendung mit Pump-Schaumspender

35 g des Mittels B wurde in einem Pump-Schaumspender vorgelegt. Unmittelbar vor der Anwendung wurden 35 g des Mittel B und 10 g des Mittels (C1) oder (C2) vorsichtig zugegeben, so dass keine Schaumbildung erfolgte. Anschließend wurde der Pump-Spender mehrfach vorsichtig auf den Kopf und wieder zurückgedreht, so dass eine gründliche Durchmischung der Komponente bei geringer Schaumbildung stattfand.

Der Schaum wurde durch Benutzung des Pumpkopfes zunächst auf die Handinnenfläche und anschließend auf den Haaransatz und die Haarlängen des Anwenders ausgebracht, zum Schluss mit einem Kamm gleichmäßig in den Fasern verteilt. Nach einer Einwirkzeit von 45 min bei Raumtemperatur wurden die Haare gründlich mit Wasser gespült, shampooniert und mit einem Haartrockner getrocknet.

## Patentansprüche

1. Verfahren zur Aufhellung keratinischer Fasern, umfassend die Schritte
i. Herstellung des anwendungsbereiten Aufhellmittels unmittelbar vor der Anwendung durch Zusammenführen
eines Mittels (A), enthaltend mindestens ein Alkalisierungsmittel,
eines Mittels (B), enthaltend mindestens ein Oxidationsmittel, und
eines Mittels (C), enthaltend mindestens ein Peroxosalz,
und anschließendes Vermischen,
ii. Ausbringung des anwendungsbereiten Aufhellmittels aus einem Schaumspendergefäß und Verteilung des anwendungsbereiten Mittel auf den Fasern,
iii. Verbleib des Aufhellmittels für einen Zeitraum von 1 bis 60 min auf den Fasern
iv. und Auswaschen aus den Fasern,
**dadurch gekennzeichnet, dass** das Mittel (A) zusätzlich mindestens ein zwitterionisches Tensid der Formel (I), 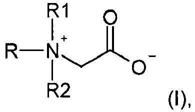 worin
R für eine gesättigte oder ungesättigte C₁₀-C₂₀-Alkylkette steht und
R1 und R2 jeweils unabhängig voneinander für eine C₁-C₄-Alkylgruppe oder eine C₂-C₄-Hydroxyalkylgruppe stehen,
enthält.

2. Verfahren nach Anspruch 1, **dadurch gekennzeichnet, dass** das Mittel (A) als Alkalisierungsmittel mindestens ein Alkanolamin enthält.

3. Verfahren nach einem der Ansprüche 1 bis 2, **dadurch gekennzeichnet, dass** das Mittel (A) ein oder mehrere zwitterionische Tenside der Formel (I) in einem Gesamtgewichtsanteil von mindestens 2,5 Gew.-%, bevorzugt von mindestens 3 Gew.-% und insbesondere bevorzugt von mindestens 4 Gew.-%, bezogen auf das Gesamtgewicht des anwendungsbereiten Aufhellmittels, enthält.

4. Verfahren nach einem der Ansprüche 1 bis 3, **dadurch gekennzeichnet, dass** das Mittel (A) zusätzlich mindestens ein nichtionisches Tensid, ausgewählt aus Alkylpolyglucosiden und/oder Alkenylpolyglucosiden und/oder ethoxylierten, nichtionischen Tensiden mit mindestens 30 Ethylenoxideinheiten, enthält.

5. Verfahren nach einem der Ansprüche 1 bis 4, **dadurch gekennzeichnet, dass** das Mittel (C) als Peroxosalz Natriumpersulfat und/oder Ammoniumpersulfat enthält.

6. Verfahren nach einem der Ansprüche 1 bis 5, **dadurch gekennzeichnet, dass** das anwendungsbereite Aufhellmittel einen Gesamttensidgehalt von mindestens 10 Gew.-%, bevorzugt mindestens 12 Gew.-%, jeweils bezogen auf das Gesamtgewicht des anwendungsbereiten Aufhellmittels, aufweist.

7. Aufhellmittel für keratinische Fasern, welches durch Vermischen aus drei getrennt voneinander konfektionierten Zubereitungen hergestellt ist, **dadurch gekennzeichnet, dass** eine der Zubereitungen ein Mittel (A) gemäß einem der Ansprüche 1 bis 4, dass eine der Zubereitungen ein Mittel (B) gemäß Anspruch 1 sowie dass eine der Zubereitungen ein Mittel (C) gemäß einem der Ansprüche 1 oder 5 ist, wobei das Mittel (A) zusätzlich mindestens ein zwitterionisches Tensid der Formel (I), 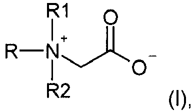 worin
R für eine gesättigte oder ungesättigte C₁₀-C₂₀-Alkylkette steht und
R1 und R2 jeweils unabhängig voneinander für eine C₁-C₄-Alkylgruppe oder eine C₂-C₄-Hydroxyalkylgruppe stehen,
enthält.

8. Mittel nach Anspruch 7, **dadurch gekennzeichnet, dass** das anwendungsbereite Aufhellmittel in Form eines Schaums vorliegt.

9. Mehrkomponentenverpackungseinheit (Kit-of-Parts) zur Aufhellung keratinischer Fasern, **dadurch gekennzeichnet, dass** es mindestens drei getrennt voneinander konfektionierte Komponenten aufweist, wobei die erste Komponente ein Mittel (A), enthaltend mindestens ein Alkalisierungsmittel, die zweite Komponente ein Mittel (B), enthaltend mindestens ein Oxidationsmittel, und die dritte Komponente ein Mittel (C), enthaltend mindestens ein Peroxosalz, enthält, **dadurch gekennzeichnet, dass** das Mittel (A) zusätzlich mindestens ein zwitterionisches Tensid der Formel (I), 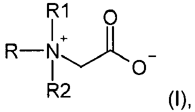 worin
R für eine gesättigte oder ungesättigte C₁₀-C₂₀-Alkylkette steht und
R1 und R2 jeweils unabhängig voneinander für eine C₁-C₄-Alkylgruppe oder eine C₂-C₄-Hydroxyalkylgruppe stehen,
enthält.

10. Mehrkomponentenverpackungseinheit nach Anspruch 9, **dadurch gekennzeichnet, dass** weiterhin ein Spendergefäß enthalten ist, das geeignet ist, die Mischung der Mittel (A), (B) und (C) in Form eines Schaums abzugeben.

## Claims

1. Method for lightening keratin fibers, comprising the following steps
i. production of the ready-to-use lightening agent immediately before application by combining an agent (A) containing at least one alkalizing agent, an agent (B) containing at least one oxidizing agent and an agent (C) containing at least one peroxo salt, and then mixing,
ii. application of the ready-to-use lightening agent from a foam dispenser vessel and distribution of the ready-to-use agent onto the fibers,
iii. leaving the lightening agent on the fibers for a period of 1 to 60 min
iv. and washing said agent out of the fibers, **characterized in that** the agent (A) additionally contains at least one zwitterionic surfactant of formula (I), 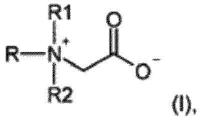 wherein
R is a saturated or unsaturated C₁₀-C₂₀ alkyl chain, and
R1 and R2 are each independently a C₁-C₄ alkyl group or a C₂-C₄ hydroxyalkyl group.

2. Method according to claim 1, **characterized in that** the agent (A) contains at least one alkanolamine as an alkalizing agent.

3. Method according to any one of claims 1 to 2, **characterized in that** the agent (A) contains one or more zwitterionic surfactants of formula (I) in a total weight proportion of at least 2.5 wt.%, preferably of at least 3 wt.% and particularly preferably of at least 4 wt.%, based on the total weight of the ready-to-use lightening agent.

4. Method according to any one of claims 1 to 3, **characterized in that** the agent (A) additionally contains at least one nonionic surfactant selected from alkyl polyglucosides and/or alkenyl polyglucosides and/or ethoxylated nonionic surfactants having at least 30 ethylene oxide units.

5. Method according to any one of claims 1 to 4, **characterized in that** the agent (C) contains sodium persulfate and/or ammonium persulfate as a peroxo salt.

6. Method according to one of claims 1 to 5, **characterized in that** the ready-to-use lightening agent has a total surfactant content of at least 10 wt.%, preferably at least 12 wt.%, based in each case on the total weight of the ready-to-use lightening agent.

7. Lightening agent for keratin fibers, which is produced by mixing three preparations packaged separately from one another, **characterized in that** one of the preparations is an agent (A) according to any one of claims 1 to 4, **in that** one of the preparations is an agent (B) according to claim 1 and **in that** one of the preparations is an agent (C) according to one of claims 1 or 5, the agent (A) additionally containing at least one zwitterionic surfactant of formula (I), 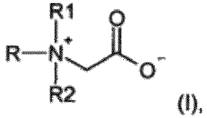 wherein
R is a saturated or unsaturated C₁₀-C₂₀ alkyl chain, and
R1 and R2 are each independently a C₁-C₄ alkyl group or a C₂-C₄ hydroxyalkyl group.

8. Agent according to claim 7, **characterized in that** the ready-to-use lightening agent is in the form of a foam.

9. Multi-component packaging unit (kit-of-parts) for lightening keratin fibers, **characterized in that** it comprises at least three separately packaged components, the first component containing an agent (A) which contains at least one alkalizing agent, the second component containing an agent (B) which contains at least one oxidizing agent, and the third component containing an agent (C) which contains at least one peroxo salt, **characterized in that** the agent (A) additionally contains at least one zwitterionic surfactant of formula (I), 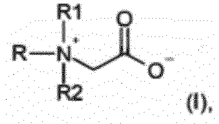 wherein
R is a saturated or unsaturated C₁₀-C₂₀ alkyl chain, and
R1 and R2 are each independently a C₁-C₄ alkyl group or a C₂-C₄ hydroxyalkyl group.

10. Multi-component packaging unit according to claim 9, **characterized in that** it further contains a dispenser vessel suitable for dispensing the mixture of agents (A), (B) and (C) in the form of a foam.

## Revendications

1. Procédé d'éclaircissement des fibres kératiniques, comprenant les étapes suivantes :
i. préparation de l'agent de blanchiment prêt à l'emploi immédiatement avant utilisation, en combinant un agent (A) contenant au moins un agent alcalinisant, un agent (B) contenant au moins un agent oxydant, et un agent (C) contenant au moins un sel peroxo, et ensuite la réalisation du mélange,
ii. application de l'agent de blanchiment prêt à l'emploi à partir d'un récipient distributeur de mousse et distribution de l'agent prêt à l'emploi sur les fibres,
iii. l'agent de blanchiment reste sur les fibres pendant une durée de 1 à 60 minutes, et
iv. lavage des fibres, **caractérisé en ce que** l'agent (A) contient en outre au moins un agent tensioactif zwittérionique de formule (I), 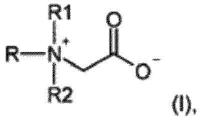 dans lequel
R représente une chaîne alkyle en C₁₀-C₂₀ saturée ou insaturée, et
R1 et R2 représentent chacun indépendamment l'un de l'autre un groupe alkyle en C₁-C₄ ou un groupe hydroxyalkyle en C₂-C₄.

2. Procédé selon la revendication 1, **caractérisé en ce que** l'agent (A) contient au moins une alcanolamine comme agent alcalinisant.

3. Procédé selon l'une des revendications 1 à 2, **caractérisé en ce que** l'agent (A) contient un ou plusieurs tensioactifs zwittérioniques de formule (I) dans une fraction pondérale totale d'au moins 2,5 % en poids, de préférence d'au moins 3 % en poids et de manière tout particulièrement préférée d'au moins 4 % en poids, par rapport au poids total de l'agent de blanchiment prêt à l'emploi.

4. Procédé selon l'une des revendications 1 à 3, **caractérisé en ce que** l'agent (A) contient en outre au moins un tensioactif non ionique choisi parmi les alkylpolyglucosides et/ou les alcénylpolyglucosides et/ou des tensioactifs non ioniques éthoxylés ayant au moins 30 unités d'oxyde d'éthylène.

5. Procédé selon l'une des revendications 1 à 4, **caractérisé en ce que** l'agent (C) contient du persulfate de sodium et/ou du persulfate d'ammonium comme sel peroxo.

6. Procédé selon l'une des revendications 1 à 5, **caractérisé en ce que** l'agent de blanchiment prêt à l'emploi présente une teneur totale en agent tensioactif d'au moins 10 % en poids, de préférence d'au moins 12 % en poids, dans chaque cas par rapport au poids total de l'agent de blanchiment prêt à l'emploi.

7. Agent de blanchiment pour fibres kératiniques, qui est obtenu par mélange de trois préparations préparées séparément l'une de l'autre, **caractérisé en ce que** l'une des préparations est un agent (A) selon l'une des revendications 1 à 4, **en ce que** l'une des préparations est un agent (B) selon la revendication 1 et **en ce que** l'une des préparations est un agent (C) selon l'une des revendications 1 ou 5, l'agent (A) étant en outre au moins un tensioactif zwitterionique de formule (I), 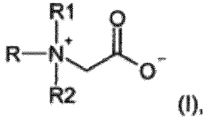 dans lequel
R représente une chaîne alkyle en C₁₀-C₂₀ saturée ou insaturée, et
R1 et R2 représentent chacun indépendamment l'un de l'autre un groupe alkyle en C₁-C₄ ou un groupe hydroxyalkyle en C₂-C₄.

8. Agent selon la revendication 7, **caractérisé en ce que** l'agent de blanchiment prêt à l'emploi se présente sous la forme d'une mousse.

9. Unité de conditionnement à composants multiples (« kit ») pour le blanchiment des fibres kératiniques, **caractérisé en ce qu'**elle comprend au moins trois composants préparés séparément, le premier composant comprenant un agent (A) contenant au moins un agent alcalinisant, le second composant comprenant un agent (B) contenant au moins un agent oxydant et le troisième composant comprenant un agent (C) contenant au moins un sel peroxo, **caractérisée en ce que** l'agent (A) comprend en outre au moins un tensioactif zwitterionique de formule (I), 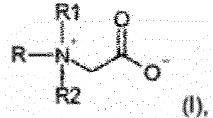 dans lequel
R représente une chaîne alkyle en C₁₀-C₂₀ saturée ou insaturée, et
R1 et R2 représentent chacun indépendamment l'un de l'autre un groupe alkyle en C₁-C₄ ou un groupe hydroxyalkyle en C₂-C₄.

10. Unité de conditionnement à composants multiples selon la revendication 9, **caractérisée en ce qu'**elle comprend en outre un récipient distributeur approprié pour distribuer le mélange des agents (A), (B) et (C) sous forme de mousse.
